# EUROPEAN PATENT APPLICATION

(11) **EP 4 850 671 A1**
(43) Date of publication of application: **09.12.2026**
(21) Application number: 25751341.6
(22) Date of filing: 24.01.2025
(51) Int. Cl.: A61K 31/437, A61P 39/00, A61P 7/10, A61P 25/00, A61P 11/00

(54) **USE OF COMPOUND IN PREPARING DRUG FOR PREVENTING AND/OR TREATING MOUNTAIN SICKNESS**

(30) Priority: 05.02.2024 CN 202410166200; 11.04.2024 CN 202410438804; 31.05.2024 CN 202410708046; 12.10.2024 CN 202411425722
(71) Applicant: Shenzhen Salubris Pharmaceuticals Co., Ltd., Shenzhen, Guangdong 518017 (CN)
(72) Inventor: XIAO, Ying, Shenzhen, Guangdong 518017 (CN); DUAN, Zhenfang, Shenzhen, Guangdong 518017 (CN); ZHANG, Ruijie, Shenzhen, Guangdong 518017 (CN); MU, Shengmei, Shenzhen, Guangdong 518017 (CN); GENG, Lei, Shenzhen, Guangdong 518017 (CN); XING, Yan, Shenzhen, Guangdong 518017 (CN)
(74) Representative: Gassner, Birgitta
(86) International application number: PCT/CN2025/074694
(87) International publication number: WO 2025/167739

(57) **Abstract**

Use of a compound and a pharmaceutical composition thereof in preparing a drug for preventing and/or treating mountain sickness. The compound is N-[7-hydroxy-5-(2-phenylethyl)[1,2,4]triazolo[1,5-a]pyridine-8-carbonyl]glycine.

## Description

### TECHNICAL FIELD

The present disclosure belongs to the technical field of pharmaceutical use, and relates to use of a compound and a pharmaceutical composition thereof in preparation of a medicament for prevention and/or treatment of mountain sickness, where the compound is *N*-[7-hydroxy-5-(2-phenylethyl)[1,2,4]triazolo[1,5-a]pyridine-8-carbonyl]glycine.

### BACKGROUND

The hypobaric and hypoxic environment at an altitude above 3,000 meters can pose serious health risks to humans, especially for individuals who have not undergone altitude acclimatization and who rapidly ascend from flatlands to high altitudes, making them highly susceptible to acute mountain sickness (AMS). Without timely treatment, life-threatening conditions such as high altitude pulmonary edema (HAPE) and high altitude cerebral edema (HACE), etc., may occur. Currently, there is a lack of relatively effective oral medications for these patients.

CN102471337B discloses a triazolopyridine compound having a prolyl hydroxylase inhibitory effect and erythropoietin production-inducing capability, specifically including a compound with a structure of the following formula:
Chemical name: N-[7-hydroxy-5-(2-phenylethyl)[1,2,4]triazolo[1,5-a]pyridine-8-carbonyl]glycine 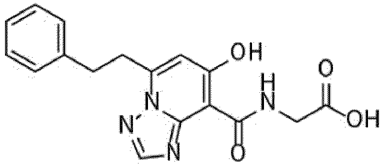
, for the treatment of anemia.

### SUMMARY

In view of the problems existing in the prior art, the present disclosure provides use of a compound in preparation of a medicament for prevention and/or treatment of mountain sickness, where the compound is N-[7-hydroxy-5-(2-phenylethyl)[1,2,4]triazolo[1,5-a]pyridine-8-carbonyl]glycine (Compound A).

The N-[7-hydroxy-5-(2-phenylethyl)[1,2,4]triazolo[1,5-a]pyridine-8-carbonyl]glycine is a compound with a structure of the following formula:
Chemical name: *N*-[7-hydroxy-5-(2-phenylethyl)[1,2,4]triazolo[1,5-a]pyridine-8-carbonyl]glycine

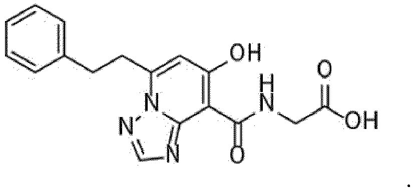

Specifically, the present disclosure provides use of a compound in preparation of a medicament for prevention and/or treatment of mountain sickness, where the compound is *N*-[7-hydroxy-5-(2-phenylethyl)[1,2,4]triazolo[1,5-a]pyridine-8-carbonyl]glycine.

In addition, the present disclosure provides use of a pharmaceutical composition comprising the compound in preparation of a medicament for prevention and/or treatment of mountain sickness, where the compound is N-[7-hydroxy-5-(2-phenylethyl)[1,2,4]triazolo[1,5-a]pyridine-8-carbonyl]glycine, and the pharmaceutical composition comprises one or more excipients.

As a preferred technical scheme of the present disclosure, it includes the use in a medicament for mountain sickness including angioedema.

As a preferred technical scheme of the present disclosure, where the mountain sickness includes acute mountain sickness.

As a preferred technical scheme of the present disclosure, where the mountain sickness includes high altitude pulmonary edema.

As a preferred technical scheme of the present disclosure, where the mountain sickness includes high altitude cerebral edema.

As a preferred technical scheme of the present disclosure, where the medicament includes an additional active ingredient for prevention and/or treatment of mountain sickness caused by hypobaric hypoxia.

As a preferred technical scheme of the present disclosure, where the daily dose is about 1 mg to about 30 mg, including:
1 mg, 1.1 mg, 1.2 mg, 1.3 mg, 1.4 mg, 1.5 mg, 1.6 mg, 1.7 mg, 1.8 mg, 1.9 mg, 2 mg, 2.1 mg, 2.2 mg, 2.3 mg, 2.4 mg, 2.5 mg, 2.6 mg, 2.7 mg, 2.8 mg, 2.9 mg, 3 mg, 3.1 mg, 3.2 mg, 3.3 mg, 3.4 mg, 3.5 mg, 3.6 mg, 3.7 mg, 3.8 mg, 3.9 mg, 4 mg, 4.1 mg, 4.2 mg, 4.3 mg, 4.4 mg, 4.5 mg, 4.6 mg, 4.7 mg, 4.8 mg, 4.9 mg, 5 mg, 5.1 mg, 5.2 mg, 5.3 mg, 5.4 mg, 5.5 mg, 5.6 mg, 5.7 mg, 5.8 mg, 5.9 mg, 6 mg, 6.1 mg, 6.2 mg, 6.3 mg, 6.4 mg, 6.5 mg, 6.6 mg, 6.7 mg, 6.8 mg, 6.9 mg, 7 mg, 7.1 mg, 7.2 mg, 7.3 mg, 7.4 mg, 7.5 mg, 7.6 mg, 7.7 mg, 7.8 mg, 7.9 mg, 8 mg, 8.1 mg, 8.2 mg, 8.3 mg, 8.4 mg, 8.5 mg, 8.6 mg, 8.7 mg, 8.8 mg, 8.9 mg, 9 mg, 9.1 mg, 9.2 mg, 9.3 mg, 9.4 mg, 9.5 mg, 9.6 mg, 9.7 mg, 9.8 mg, 9.9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, and 30 mg.

As an implementation technical scheme of the present disclosure, the preparation of the compound is incorporated herein by reference to the technical scheme recorded in CN201780072928.2.

The crystal form and preparation used in the pharmaceutical composition are incorporated herein by reference to the technical scheme recorded in US20200017492A1.

The composition further includes other active pharmaceutical agents (including additional active ingredients), the active pharmaceutical agents include, but are not limited to, anti-inflammatory agents, antiemetics, diuretics, calcium antagonists, bronchodilators, antipyretic analgesics, vasodilators, and adrenocorticoids.

The pharmaceutical composition of the present disclosure includes one or more accessories (excipients), including but not limited to, solvents, propellants, solubilizers, cosolvents, emulsifiers, colorants, binders, disintegrants, fillers, lubricants, wetting agents, osmotic pressure regulators, stabilizers, glidants, flavoring agents, preservatives, suspending agents, coating materials, flavors, anti-adherents, chelating agents, penetration enhancers, pH adjusters, buffers, plasticizers, surfactants, foaming agents, defoamers, thickeners, inclusion agents, humectants, absorbents, diluents, flocculants and deflocculants, filter aids, and release retardants.

The dosage form of the pharmaceutical composition of the present disclosure is selected from the group consisting of tablets, capsules, injections, sprays, aerosols, nasal drops, powder sprays, suppositories, patches, and gels, etc.

Preferably, the tablets are selected from the group consisting of conventional tablets, immediate-release tablets, sustained-release tablets, controlled-release tablets, film-coated tablets, sugar-coated tablets, buccal tablets, sublingual tablets, and bioadhesive tablets; the capsules are selected from the group consisting of hard capsules and soft capsules; the injections are selected from the group consisting of sterile or bacteriostatic aqueous injections, oily injections, freeze-dried powder injections, and microspheres for injection; the sprays are selected from the group consisting of oral sprays, nasal sprays, and topical skin sprays; the aerosols are selected from the group consisting of pulmonary inhalation aerosols and topical skin aerosols; the nasal drops are selected from the group consisting of nasal drop solutions and nasal drop gels; and the powder sprays are selected from the group consisting of oral powder sprays, nasal powder sprays, and topical skin powder sprays.

The use of the pharmaceutical composition of the present disclosure in a medicament for prevention and/or treatment of mountain sickness includes, but is not limited to, the use for treatment and/or prevention of acute mountain sickness, including high altitude cerebral edema, pulmonary edema, myocardial ischemia, angina pectoris, cerebral circulation insufficiency, chronic obstructive pulmonary disease, sleep apnea syndrome, cerebral infarction, myocardial infarction, degenerative disease, and impaired operational capacity in high-altitude, deep-water, or underground confined spaces, etc., caused by hypoxia.

The beneficial effects of the present disclosure over the prior art include but are not limited to:
(1) use of the compound of the present disclosure in preparation of a medicament for treatment of mountain sickness is superior in terms of lower dosage and reduced adverse effects, etc.
(2) Under the same dosage and administration frequency, the compound of the present disclosure exhibits superior efficacy in a hypoxic environment compared to Roxadustat.
(3) The compound of the present disclosure has both good preventive and therapeutic effects in a hypoxic environment.

### BRIEF DESCRIPTION OF DRAWINGS

FIG. 1 shows survival time in mice of each group in Example 1 of the present disclosure.
FIG. 2 shows improvements in RBC and HCT in mice of each group in Example 1 of the present disclosure.
   Note: In FIG. 1 and FIG. 2, **P < 0.01 compared with the vehicle group.
FIG. 3 shows the survival time of mice in a sodium nitrite poisoning experiment of Example 2 of the present disclosure (mean ± SEM; ***p < 0.001 vs a vehicle group; ##p < 0.01 vs a Roxadustat group).
FIG. 4 shows the effect of pre-hypoxia administration on plasma EPO levels in Example 3 of the present disclosure.
FIG. 5 shows the effect of pre-hypoxia administration on the left ventricular function of mice in Example 3 of the present disclosure.
FIG. 6 shows the effect of concurrent administration with hypoxia induction on plasma EPO levels in Example 4 of the present disclosure.
FIG. 7 shows the effect of concurrent administration with hypoxia induction on the left ventricular function of mice in Example 4 of the present disclosure.

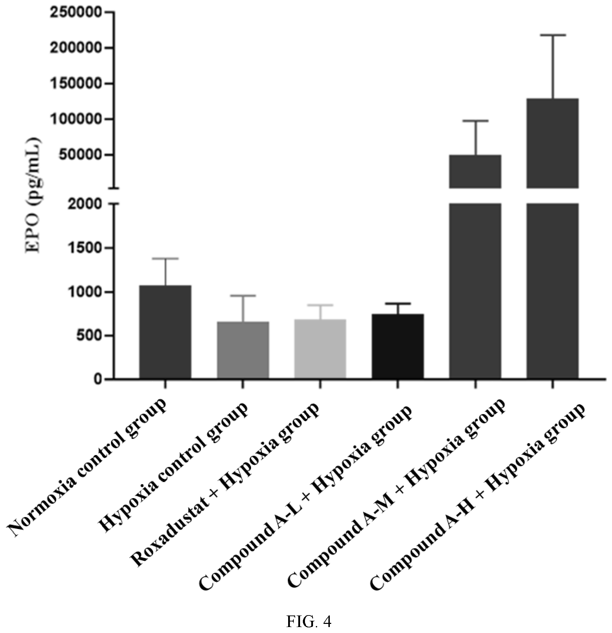

Note: In FIG. 4 to FIG. 7, *P < 0.05, and ****P < 0.0001.

### DETAILED DESCRIPTION

The present disclosure will be further described in detail hereafter in combination with examples, but the embodiments of the present disclosure are not limited thereto.

### Example 1: Evaluation via Normobaric Closed Hypoxia Tolerance Experiment

Experimental Methods: male BALB/c mice were used. After acclimatization, the mice were randomly divided into groups based on body weights. The groups were as follows: a vehicle control group, a Compound A low-dose group (50 mg/kg), a Compound A high-dose group (100 mg/kg), a Roxadustat low-dose group (50 mg/kg), and a Roxadustat high-dose group (100 mg/kg), with 12 mice per group. The vehicle or drug was administered by gavage once daily for 7 consecutive days. Blood samples were collected on Day 4 of administration to measure red blood cell (RBC) count and hematocrit (HCT). The hypoxia tolerance test was conducted 1 h after the final administration.

Hypoxia Tolerance Test Indicators: at room temperature, mice were individually placed into 250 ml wide-mouth bottles containing 5 g of soda lime (wrapped in double-layer gauze). Vaseline was applied to the bottle stoppers and rims to prevent air leakage. After the animals were placed in the bottles, the stoppers were tightened and timing commenced immediately. A death endpoint was defined as the cessation of breathing and the absence of leg twitching. The survival time of mice in each group was recorded. Experimental data were expressed as mean ± standard deviation (SD) and subjected to statistical analysis.

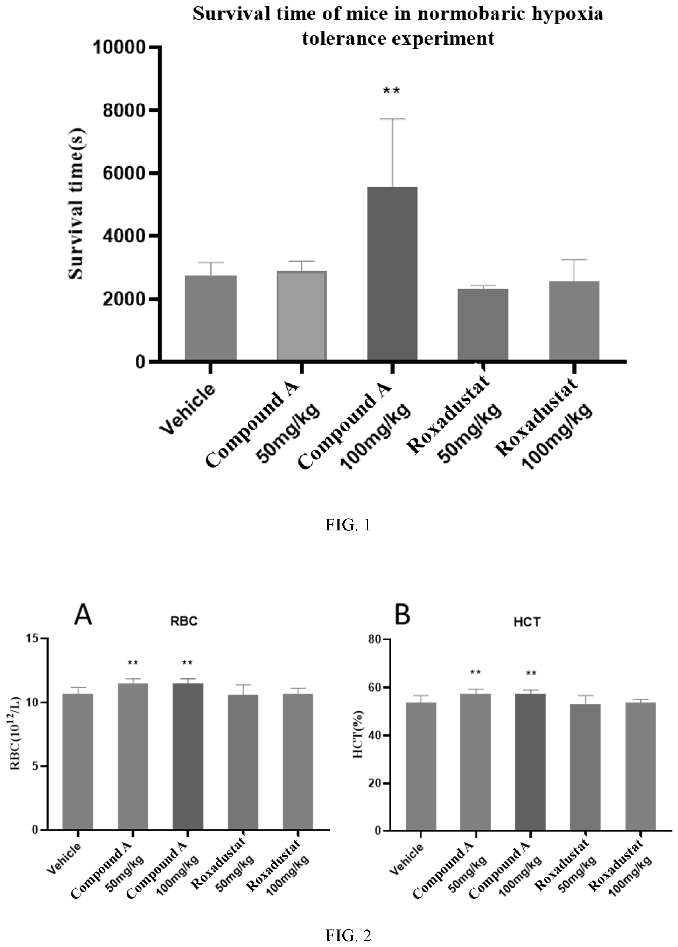

Experimental Results: compared with the vehicle control group, the survival time of the mice in each Compound A administration group was significantly prolonged in a dose-dependent manner, and the survival time was significantly prolonged at high doses. However, Roxadustat did not exhibit such effects at the same dose, as shown in FIG. 1.

Meanwhile, Compound A significantly increased red blood cell (RBC) count and hematocrit (HCT) in both the low-dose and high-dose groups, and the improvement was superior to that of Roxadustat, as shown in FIG. 2.

In conclusion, under the same dosage and administration frequency, Compound A exhibited improved erythropoietic effects and hypoxia tolerance enhancement compared to Roxadustat.

### Example 2: Sodium Nitrite-Induced Toxic Hypoxia Experiment in Mice

Experimental Methods: male Balb/c mice were used. After quarantine and acclimatization, the mice were randomly divided into groups based on body weights. The groups were as follows: a vehicle control group (0.5% CMC-Na), a Compound A administration group (100 mg/kg/day), and a Roxadustat administration group (100 mg/kg/day), with 10 mice per group. The vehicle or drug was administered by gavage once daily for 7 consecutive days. Prior to a 4^{th} administration, 100 µL of whole blood was collected via retro-orbital bleeding and placed in EDTA-K2 anticoagulant tubes, and the red blood cell (RBC) count, hemoglobin (Hb), and hematocrit (Hct) were measured using a hematology analyzer. One hour after the final gavage, mice in all groups were intraperitoneally injected with sodium nitrite at a dose of 200 mg/kg, and timing commenced immediately after the injection. A death endpoint was defined as the cessation of breathing of the mice (sudden flaccidity after twitching and the absence of chest movement). The time from sodium nitrite administration to cessation of breathing of the mice (i.e., the survival time) was recorded.

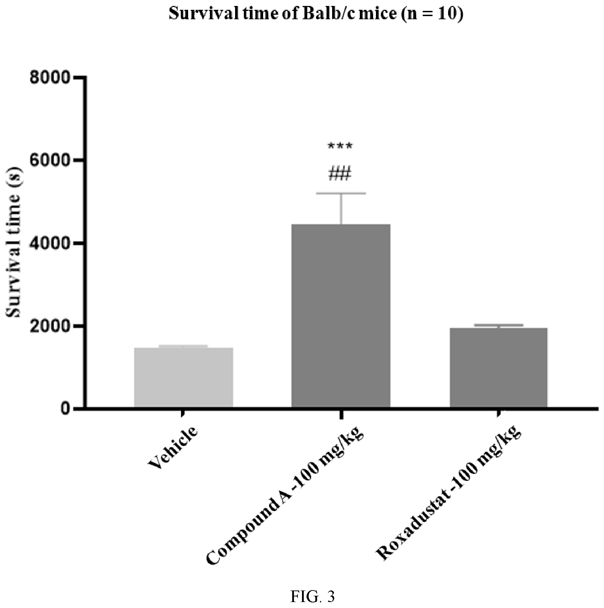

The results were shown in FIG. 3 and Table 1:

**Table 1: Hematological Indicators of Mice on Day 4 of Administration**

| Groups | Day 4 Haematology | | |
|---|---|---|---|
| | RBC (10¹²/L) | Hb (g/L) | Hct (%) |
| Vehicle | 10.07 ± 0.36 | 159.6 ± 4.7 | 48.82 ± 1.82 |
| Compound A-100 mg/kg | 10.64 ± 0.24 | 176.6 ± 3.2 | 51.57 ± 0.89 |
| Roxadustat-100 mg/kg | 10.2 ± 0.27 | 173.4 ± 4.35 | 49.8 ± 1.45 |

Compared with the vehicle control group, Compound A (100 mg/kg/day) significantly prolonged the survival time of the mice following sodium nitrite poisoning. Hematological analysis results showed that after four consecutive days of administration of Compound A or Roxadustat, respectively, RBC, Hb, and Hct levels were elevated compared to the vehicle group, with a more pronounced improvement observed in the Compound A group.

This suggests that that under the same dosage and administration frequency, Compound A exhibited superior erythropoietic effects and hypoxia tolerance to sodium nitrite-induced toxic hypoxia compared to Roxadustat.

### Example 3: Effects of Administration Prior to Hypoxia Induction in Mice (Prophylactic Administration)

Experimental Methods: male C57 mice were used. After a 3-day acclimatization, the mice were divided into a normoxia control group, a hypoxia control group, a Roxadustat (60 mg/kg) + hypoxia group, a Compound A low-dose (15 mg/kg) + hypoxia group, a Compound A medium-dose (30 mg/kg) + hypoxia group, and a Compound A high-dose (60 mg/kg) + hypoxia group based on body weights. For the first three days of the experiment, all groups were maintained in a normoxic environment and administered vehicle or drug once daily. Starting from Day 4, all animals except those in the normoxia control group were placed into a hypobaric hypoxia chamber simulating an altitude of 5,000 meters, and were administered continually until Day 10. The animals were weighed daily throughout the experiment. Echocardiography was performed one day prior to the experimental endpoint, and blood gas analysis and hematological measurements were conducted at the experimental endpoint.

Experimental Results: as shown in FIG. 4, EPO assay results indicated that at 24 h after the final administration, EPO levels in the Compound A medium- and high-dose groups remained at high levels, demonstrating superiority over Roxadustat at equivalent doses.

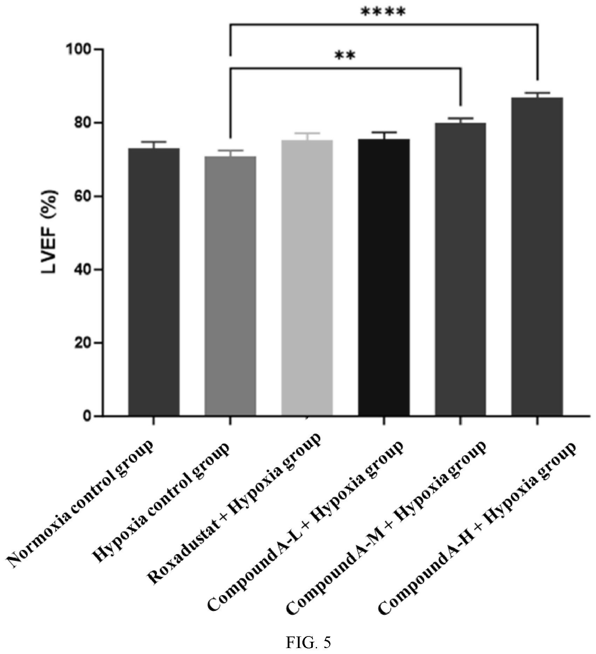

As shown in FIG. 5, echocardiography showed that left ventricular ejection fraction (LVEF) in mice in the hypoxia group exhibited a decreasing trend compared to the normoxia control group, suggesting potential impairment of left ventricular function. Compared with the hypoxia group, Compound A administration improved LVEF in a dose-dependent manner, demonstrating superiority over Roxadustat at equivalent doses.

### Example 4: Effects of Compound A Administered Concurrently with Hypoxia Induction in Mice

Experimental Methods: male C57 mice were used. After a 3-7 day acclimatization, the mice were randomly divided into a normoxia control group, a hypoxia control group, a Roxadustat (100 mg/kg) + hypoxia group, and a Compound A high-dose (100 mg/kg) + hypoxia group based on body weights. Starting from Day 1, the mice in the hypoxia groups were placed in a hypobaric hypoxia chamber simulating an altitude of 5,000 meters, while the mice in the normoxia control group were maintained in a normoxic environment. All groups were administered vehicle or drug once daily for 7 consecutive days. The mice were weighed daily throughout the experiment, and hematological tests and echocardiography were performed at the experimental endpoint.

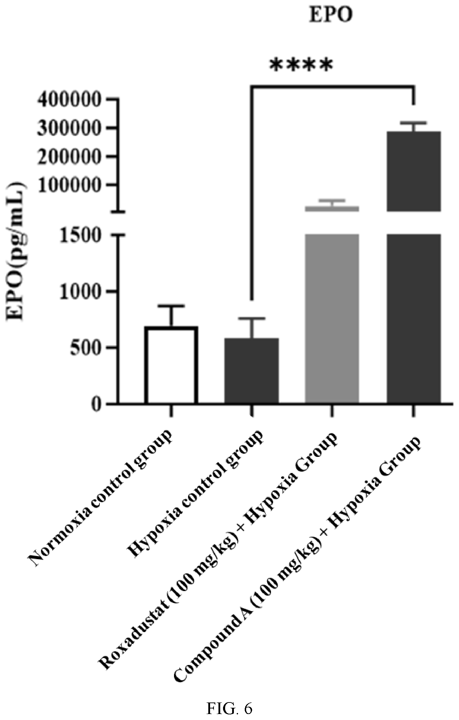

Results: as shown in FIG. 6, plasma EPO assay results showed that the Compound A group exhibited a greater increase in EPO concentration than the Roxadustat group at the equivalent dose.

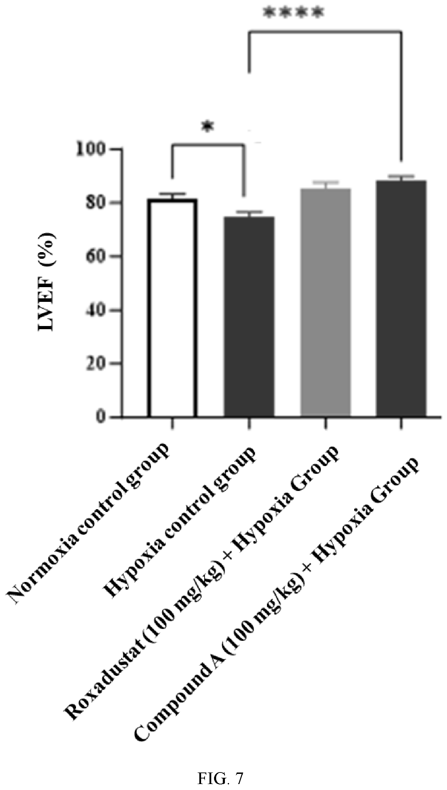

As shown in FIG. 7, echocardiography showed that the left ventricular ejection fraction (LVEF) of mice in the Compound A group showed a significant increase compared to the hypoxia group, and this increase was greater than that in the Roxadustat group.

The aforementioned examples are preferred embodiments of the present disclosure; however, the embodiments of the present disclosure are not limited thereto. Any other changes, modifications, substitutions, combinations, or simplifications made without departing from the essence and principles of the present disclosure shall be equivalent substitutions and be included in the scope of protection of the present disclosure.

## Claims

1. Use of a compound in preparation of a medicament for prevention and/or treatment of mountain sickness, wherein the compound is *N*-[7-hydroxy-5-(2-phenylethyl)[1,2,4]triazolo[1,5-a]pyridine-8-carbonyl]glycine.

2. Use of a pharmaceutical composition comprising a compound in preparation of a medicament for prevention and/or treatment of mountain sickness, wherein the compound is *N*-[7-hydroxy-5-(2-phenylethyl)[1,2,4]triazolo[1,5-a]pyridine-8-carbonyl]glycine, and the pharmaceutical composition comprises one or more excipients.

3. The use according to claim 1 or 2, comprising the use in a medicament for mountain sickness comprising angioedema.

4. The use according to claim 1 or 2, wherein the mountain sickness comprises acute mountain sickness.

5. The use according to claim 1 or 2, wherein the mountain sickness comprises high altitude pulmonary edema.

6. The use according to claim 1 or 2, wherein the mountain sickness comprises high altitude cerebral edema.

7. The use according to claim 1 or 2, wherein the medicament comprises an additional active ingredient for prevention and/or treatment of mountain sickness caused by hypobaric hypoxia.
